# EUROPEAN PATENT APPLICATION

(11) **EP 2 889 744 A1**
(43) Date of publication of application: **01.07.2015**
(21) Application number: 14195178.0
(22) Date of filing: 27.11.2014
(51) Int. Cl.: G06F 3/0484, A61B 8/00, G06F 19/00

(54) **Method and apparatus for displaying medical images**

(30) Priority: 09.12.2013 KR 20130152649
(71) Applicant: Samsung Electronics Co., Ltd, Gyeonggi-do 443-742 (KR)
(72) Inventor: Kim, Hei-soog, Gyeonggi-do (KR); Cho, Hyug-rae, Seoul (KR); Park, Seon-mi, Gyeonggi-do (KR); Shin, Je-yong, Gyeonggi-do (KR)
(74) Representative: Jacobs, Bart

(57) **Abstract**

A method of displaying medical images includes displaying a medical image list on a display; changing a size of a first medical image included in the medical image list according to a user input; changing respective sizes of medical images other than the first medical image in the medical image list according to the changed size of the first medical image; determining a layout of the medical image list according to the changed size of the first medical image and a size of the display; and aligning the medical image list according to the determined layout.

## Description

Apparatuses and methods consistent with exemplary embodiments relate to displaying at least one medical image included in a medical image list.

A medical image may be used to diagnose diseases and provide treatment for patients. Along with the development of medical imaging technologies, various types of medical imaging methods are being developed. Therefore, there is a need for a viewer system that allows a user to effectively compare various medical images.

In a related art medical image displaying method, a medical image may be displayed in a 1x1 array, i.e., only one medical image is shown on a screen, or displayed in a 1x2 matrix so that a viewer may compare two medical images to each other. Alternatively, the viewer may display medical images in one of various default matrices that are provided by a manufacturer so that the user may compare a plurality of medical images at once.

Exemplary embodiments address at least the above problems and/or disadvantages and other disadvantages not described above. Also, the exemplary embodiments are not required to overcome the disadvantages described above, and may not overcome any of the problems described above.

One or more exemplary embodiments provide a method and an apparatus for displaying medical images of a medical image list, in which a layout of the medical image list may be changed according to a change in a size of at least one medical image in the medical image list.

According to an aspect of an exemplary embodiment, a method of display medical images includes displaying a medical image list on a display; changing a size of a first medical image included in the medical image list according to a user input; changing respective sizes of other medical images excluding the first medical image in the medical image list, according to the changed size of the first medical image; determining a layout of the medical image list according to the changed size of the first medical image and a size of the display; and aligning the medical image list according to the determined layout.

The changing of the size of the first medical image included in the medical image list according to the user input may include receiving a selection input of a predetermined area of the first medical image and a drag input of a selected predetermined area.

The changing of the respective sizes of the other medical images may include adjusting the respective sizes of the other medical images to be the same as the size of the first medical image.

The changing of the size of the first medical image may include changing an aspect ratio of the first medical image according to the user input.

The changing of the size of the first medical image may include changing a size of a text associated with the first medical image.

The text associated with the first medical image may include at least one selected from patient information, information regarding a medical image apparatus that has acquired the first medical image, scan environment information of the first medical image, a description of the first medical image, and annotation information.

The determining of the layout may include determining the number of medical images to be displayed on the display, according to the changed size of the first medical image and the size of the display.

Adjusting the changed size of the first medical image and the respective sizes of the other medical images according to the determined number of medical images may be further included.

The determining of the layout may include determining the number of pages of the medical list according to the determined number of medical images.

The determining of the layout may include determining a size of a blank area according to the changed size and the aspect ratio of the first medical image.

The determining of the layout may include determining a display location of a text associated with the first medical image.

The determining of the layout may include adjusting an amount of a text displayed on the display.

According to an exemplary embodiment, an apparatus for displaying medical images includes a display configured to display a medical image list; a user input unit configured to receive a user input for changing a size of a first medical image included in the medical image list; and a controller configured to change the size of the first medical image according to the user input, change respective sizes of other medical images excluding the first medical image in the medical image list according to the changed size of the first medical image, and determine a layout of the medical image list according to the changed size of the first medical image and a size of the display, and aligning the medical image list according to the determined layout.

The user input unit may receive a selection input of a predetermined area of the first medical image and a drag input of a selected predetermined area as the user input.

The controller may adjust the respective sizes of the other medical images to be the same as the size of the first medical image.

The controller may change an aspect ratio of the first medical image according to the user input.

The controller may change a size of a text associated with the first medical image.

The controller may determine the number of medical images to be displayed on the display, according to the changed size of the first medical image and the size of the display.

The controller may adjust the changed size of the first medical image and the respective sizes of the other medical images according to the determined number of medical images.

The controller may determine the number of pages of the medical list according to the determined number of medical images.

The controller may determine a size of a blank area according to the changed size and the aspect ratio of the first medical image.

The controller may determine a display location of a text associated with the first medical image, according to the changed size of the first medical image and the size of the display.

The controller may adjust an amount of a text displayed on the display.

According to an aspect of an exemplary embodiment, an apparatus for displaying a medical image list including medical images includes: a display configured to display the medical images included in the medical image list; a user input unit configured to receive a user input for changing a size of a first medical image, among the medical images, to a first size, and a controller configured to determine a second size of the first medical image by adjusting the first size according to the user input based on a size of the display, wherein the controller is configured to control the display to display the medical images including the first medical image in the second size.

The controller may determine the second size having a first height and a first width such that at least one of a second height and a second width of the display is substantially an integer multiple of at least one of the first height and the first width.

The controller may determine a font size of a character included in the first medical image according to the second size and controls the display to display the character in the determined font size.

The above and/or other aspects will be more apparent by describing certain exemplary embodiments, with reference to the accompanying drawings, in which:
FIG. 1 is a diagram illustrating a medical image display apparatus according to an exemplary embodiment;
FIG. 2 is a view illustrating a method of providing a plurality of medical images according to an exemplary embodiment;
FIG. 3 is a flowchart of a method of displaying medical images, according to an exemplary embodiment;
FIGS. 4A, 4B, and 4C illustrate examples in which a layout of a medical image list changes according to an enlarged size of a first medical image;
FIGS. 5A, 5B, and 5C illustrate examples in which the layout of a medical image list is changed according to a reduced size of a first medical image;
FIGS. 6A, 6B, and 6C illustrating adjusting a size of at least one medical image included in a medical image list, according to an exemplary embodiment;
FIGS. 7A, 7B, 7C, and 7D illustrate an example in which a layout of a medical image list changes according to an aspect ratio of a first medical image; and
FIGS. 8 and 9 are block diagrams of a medical image display apparatus, according to an exemplary embodiment.

Exemplary embodiments are described in greater detail below with reference to the accompanying drawings.

In the following description, like drawing reference numerals are used for like elements, even in different drawings. The matters defined in the description, such as detailed construction and elements, are provided to assist in a comprehensive understanding of the exemplary embodiments. However, it is apparent that the exemplary embodiments can be practiced without those specifically defined matters. Also, well-known functions or constructions are not described in detail since they would obscure the description with unnecessary detail.

It will be understood that the terms "comprises" and/or "comprising" used herein specify the presence of stated features or components, but do not preclude the presence or addition of one or more other features or components. In addition, the terms such as "unit", "-er (-or)", and "module" described in the specification refer to an element for performing at least one function or operation, and may be implemented in hardware, software, or the combination of hardware and software.

In the present specification, the term "image" may refer to multi-dimensional data composed of discrete image elements (e.g., pixels in a two-dimensional image and voxels in a three-dimensional image).

Furthermore, in the present specification, the term "object" may include a person or an animal, or a part of a person or an animal. For example, the "object" may include organs such as, for example, a liver, a heart, a womb, a brain, a breast, an abdomen, or a blood vessel. Furthermore, the "object" may include a phantom. The phantom is a material having a volume that is approximately a density and effective atomic number of a living body.

Furthermore, in the present specification, the term "user" refers to a medical professional, such as a doctor, a nurse, a medical laboratory technologist, a radiologist, and a sonographer, but not limited thereto.

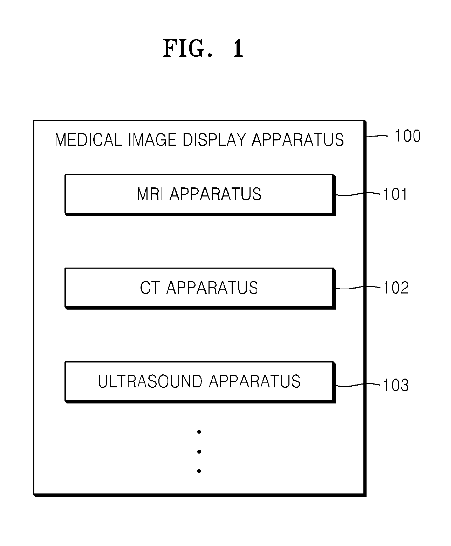

FIG. 1 is a diagram illustrating a medical image display apparatus 100 according to an exemplary embodiment.

According to an exemplary embodiment, the medical image display apparatus 100 may be an apparatus that acquires medical images and displays the acquired medical images on a screen. For example, the medical image display apparatus 100 may include at least one of a magnetic resonance imaging (MRI) apparatus 101, a computer tomography (CT) apparatus 102, an X-ray imaging apparatus (not shown), an angiography apparatus (not shown), and an ultrasound apparatus 103, but is not limited thereto.

The MRI apparatus 101 is capable of acquiring a sectional image of a part of an object by expressing, in a contrast comparison, a strength of an MR signal with respect to a radio frequency (RF) signal generated in a magnetic field having a specific strength.

Since the CT apparatus 102 is capable of providing a cross-sectional image of the object, the CT apparatus 102 may express internal structures (e.g., organs such as kidneys and lungs) of the object without an overlap therebetween, compared to a general X-ray imaging apparatus. The CT apparatus 102 may obtain a plurality of pieces of image data with a thickness, e.g., not more than 2 mm by performing several tens to several hundreds of times scans per second and may process the plurality of pieces of image data. Accordingly, the CT apparatus 102 may provide a relatively accurate cross-sectional image of the object.

The X-ray imaging apparatus is capable of acquiring images of internal structures of the human body by transmitting X-rays through a human body. The angiography apparatus is an apparatus that enables a user to look at a blood vessel (e.g., an artery or a vein) of an object by injecting a contrast medium thereto through a thin tube, which is called a catheter, having a diameter of about 2 mm.

The ultrasound apparatus 103 transmits ultrasound signals from a surface of the body of the object to a predetermined part of the body of the object, and acquires images of blood streams or sections of soft tissues by using ultrasound signals that are reflected from the predetermined part of the body.

According to an exemplary embodiment, the medical image display apparatus 100 may be implemented in various ways. For example, the medical image display apparatus 100 may be implemented as a fixed terminal, or a mobile terminal such as a smartphone, a laptop computer, a personal digital assistant (PDA), and a tablet personal computer (PC).

According to an exemplary embodiment, the medical image display apparatus 100 may transmit or receive medical image data to or from a hospital server or another medical apparatus in a hospital connected through a picture archiving and communication system (PACS), and perform data communication according to the digital imaging and communications in medicine (DICOM) standard.

According to an exemplary embodiment, the medical image display apparatus 100 may include a touch screen. The touch screen may detect locations, areas, and/or pressure of touch inputs. Also, the touch screen may detect not only a physical touch but also a proximity touch.

In the exemplary embodiments, the term "physical touch" refers to an actual touch received via a touch screen using a touch tool (e.g., a finger and/or an electronic pen), and the term "proximity touch" refers to when the touch tool approaches the touch screen within a predetermined distance therefrom.

According to an exemplary embodiment, the medical image display apparatus 100 may detect touch gestures of the user on the medical images by using the touch screen. The touch gestures (or touch inputs) of the user may include, for example but not limited to, tapping, touch and hold, double tapping, dragging, panning, flicking, drag and drop, swiping, pinching, and the like.

According to an exemplary embodiment, the medical image display apparatus 100 may provide a part of or all buttons for controlling the medical image list in a graphical user interface (GUI).

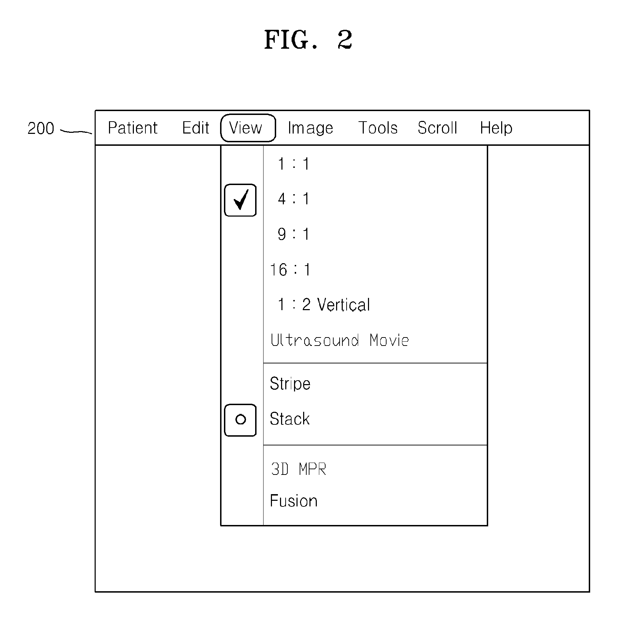

As illustrated in FIG. 2, the medical image display apparatus 100 may provide a layout setting window 200 for displaying a plurality of medical images. The user may select a layout from among layouts included in "View" menu of the layout setting window 200. For example, when the user selects "4:1" from the "View" menu, four medical images may be displayed on a screen. While the four medical images are displayed on the screen, when the user wants to display nine medical images on the screen, the user may select "9:1" from the "View" menu of the layout setting window 200.

When the user wants to display ten medical images on a screen, since "10:1" is not included in the "View" menu of the layout setting window 200, the user may select "9:1" or "16:1." Therefore, a GUI that allows the user to freely change a layout of the medical image list by a simple operation is needed.

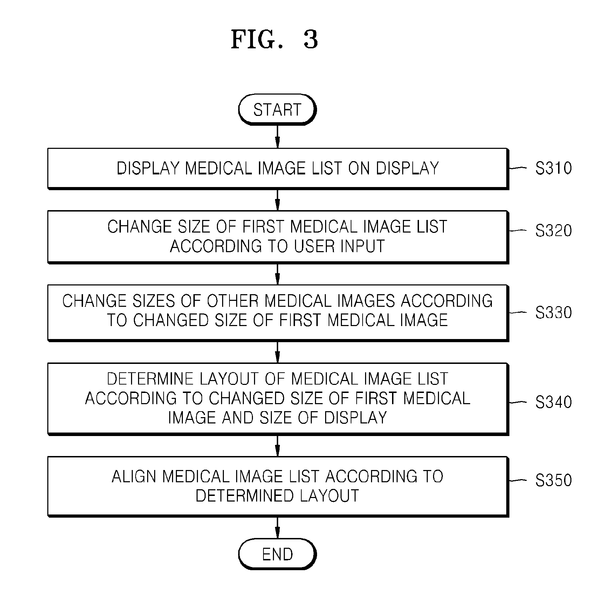

Hereinafter, a medical image display method of the medical image display apparatus 100 that adaptively changes the layout of the medical image list when the user changes a size of a medical image included in the medical image list will be described with reference to FIG. 3.

FIG. 3 is a flowchart of a method for displaying medical images, according to an exemplary embodiment.

In operation S310, the medical image display apparatus 100 may display the medical image list on a display. According to an exemplary embodiment, the display may indicate an apparatus, a space, or an area in which the medical images are displayed.

According to an exemplary embodiment, the medical image list may include at least one medical image. The medical images in an exemplary embodiment may include various images. For example, the medical images may include an MR image, a CT image, an ultrasound image, and an X-ray image, but are not limited thereto. The ultrasound images may include, but are not limited to, a brightness mode (B-mode) image using brightness to represent an amplitude of ultrasound echo signals reflected from an object, a color Doppler image using a Doppler effect to represent a velocity of a moving object by colors, a spectral Doppler image using the Doppler effect to represent images of a moving object in a spectrum form, a motion mode (M-mode) image representing motions of an object over time at a specific location, and an elastic mode image representing different reactions of an object when compression is and is not applied to the object. Also, the medical images may include two-dimensional (2D) images, three-dimensional (3D) images, and four-dimensional (4D) images, but are not limited thereto. Hereinafter, an example of a 2D MR image will be described for the convenience of description.

According to an exemplary embodiment, the medical image display apparatus 100 may receive at least one medical image selected by the user from a memory, and generate a medical image list.

According to an exemplary embodiment, the medical image display apparatus 100 may receive a medical image selected by the user from an external source, and generate a medical image list that includes the received medical image.

In operation S320, the medical image display apparatus 100 may change a size of a first medical image included in the medical image list according to a user input.

According to an exemplary embodiment, the medical image display apparatus 100 may receive a selection input in a predetermined area of the first medical image and a drag input in the predetermined area. For example, the user may click and drag the predetermined area of the first medical image. Alternatively, when the display is a touch screen, the user may touch and drag the predetermined area of the first medical image by using a touch tool (e.g., a finger and/or an electronic pen). In this case, the medical image display apparatus 100 may increase or reduce the size of the first medical image according to at least one of, for example, a direction of the drag input, a length of the drag input, and a finishing location or a time point of the drag input.

According to an exemplary embodiment, the predetermined area of the first medical image is a portion in the first medical image that may be adjusted in a size. For example, the predetermined area of the first medical image may be a peripheral area or a central area of the first medical image.

According to an exemplary embodiment, the medical image display apparatus 100 may change an aspect ratio of the first medical image according to the user input. The aspect ratio of the first medical image refers to a width to height ratio of the first medical image.

According to an exemplary embodiment, when the user selects (e.g., clicks or touches) and drags the predetermined area of the first medical image, the medical image display apparatus 100 may increase or reduce the aspect ratio of the first medical image according to, for example, a direction or a length of the dragging. For example, when the user touches and drags the first medical image to a right direction, a width of the first medical image increases, and thus, the aspect ratio of the first medical image may increase.

According to an exemplary embodiment, the medical image display apparatus 100 may change a size of a text associated with the first medical image according to a change in the size of the first medical image. According to an exemplary embodiment, the medical image display apparatus 100 may change the size of the text associated with the first medical image according to a size change ratio of the first medical image. For example, when the size of the first medical image is reduced or increased, the size of the text may also be reduced or increased.

According to an exemplary embodiment, the text associated with the first medical image may include at least one of, for example, patient information (e.g., a patient identification (ID) or object information), information regarding the medical image apparatus that has acquired the first medical image (e.g., a type, a name, or a location of the medical image apparatus), scan environment information of the first medical image (e.g., repetition time (TR) or echo time (TE)), a description of the first medical image (e.g., image brightness level), and annotation information, but is not limited thereto.

In operation S330, the medical image display apparatus 100 may change respective sizes of medical images other than the first medical image in the medical image list, according to the changed size of the first medical image.

For example, the medical image display apparatus 100 may adjust respective sizes of the other medical images to be substantially the same as the size of the first medical image. Also, the medical image display apparatus 100 may adjust respective aspect ratios of the other medical images to be substantially the same as the aspect ratio of the first medical image.

According to an exemplary embodiment, the medical image display apparatus 100 may adjust respective sizes of texts associated with the other medical images according to the changed size of the text associated with the first medical image.

In operation S340, the medical image display apparatus 100 may determine a layout of the medical image list according to the changed size of the first medical image and a size of the display.

According to an exemplary embodiment, the medical image display apparatus 100 may determine the number of medical images to be displayed on the display according to the changed size of the first medical image and the size of the display. For example, when the size of the first medical image is 2"x2" and the size of the display is 10"x8", the medical image display apparatus 100 may determine to display 20 medical images on the display.

According to an exemplary embodiment, the medical image display apparatus 100 may determine the number of pages of the medical image list according to the determined number of medical images. For example, when sixty medical images are included in the medical image list and twenty medical images are determined to be displayed on the display, the medical image display apparatus 100 may determine that 3 pages are included in the medical image list.

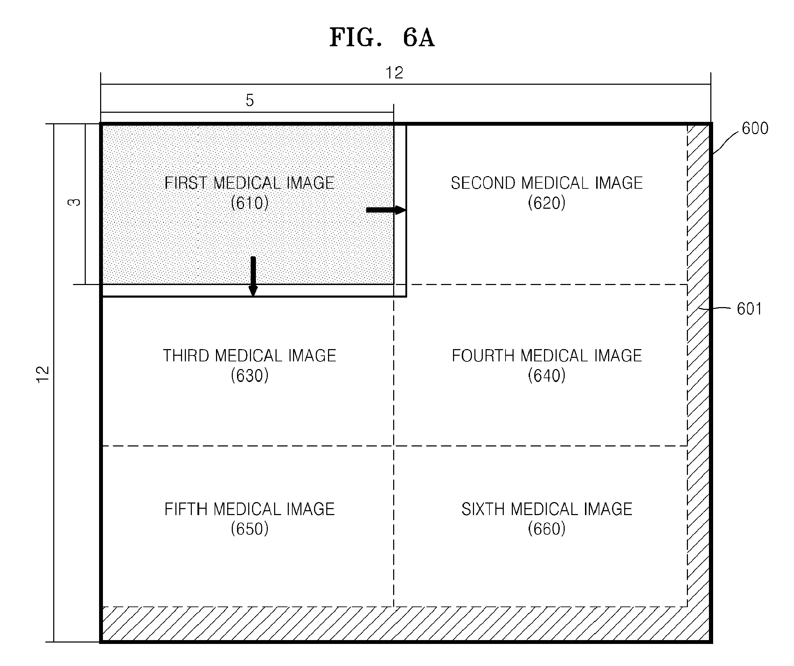

According to an exemplary embodiment, the medical image display apparatus 100 may adjust the changed size of the first medical image and the respective sizes of the other medical images according to the determined number of medical images. For example, the medical image display apparatus 100 may adjust the changed size of the first medical image so that the number of medical images displayed on the display is an integer. The adjustment of the size of the first medical image by the medical image display apparatus 100 will be described below with reference to FIGS. 6A to 6C.

According to an exemplary embodiment, the medical image display apparatus 100 may determine a size of a blank area of the display according to at least one of the changed size and the aspect ratio of the first medical image. The blank area may indicate an area of the display in which the first medical image and the other medical images are not displayed.

According to an exemplary embodiment, the medical image display apparatus 100 may determine a display location of the text of the first medical image. For example, the medical image display apparatus 100 may display the text of the first medical image such that the text is on the first medical image or overlaps at least a portion of the first medical image, according to at least one of the changed size and the aspect ratio of the first medical image. The medical image display apparatus 100 may extract a non-interest area (e.g., an area in which an object of interest is not displayed) from the first medical image, and display the text of the first medical image on the non-interest area.

Alternatively, the medical image display apparatus 100 may display the text of the first medical image on the blank area, according to at least one of the changed size and the aspect ratio of the first medical image. For example, when upper and lower blank areas of the first medical image are greater than left and right blank areas thereof, the medical image display apparatus 100 may display the text of the first medical image on the upper and lower blank areas.

According to an exemplary embodiment, the medical image display apparatus 100 may adjust an amount of the text displayed on the display according to the changed size of the first medical image or the number of medical images displayed on the display. For example, the medical image display apparatus 100 may reduce an amount of the text of the first medical image when there are many medical images are displayed on the display, or when the changed size of the first medical image is relatively small. As another example, when the display is displaying twenty or more medical images, the medical image display apparatus 100 may display a text having less than five lines on the display according to a priority of texts. When the display is displaying less than five medical images, the medical image display apparatus 100 may display a text having up to ten lines.

In operation S350, the medical image display apparatus 100 may arrange the medical image list according to the determined layout. The determined layout may include the number of medical images to be displayed on a page, a total number of pages of the medical image list, a display location of a text of at least one medical image included in the medical image list, an amount of a text to be displayed, and a size of the blank area, but are not limited thereto.

For example, according to the determined layout, the medical image display apparatus 100 may display two medical images on a page, and texts that respectively correspond to the two medical images on the blank area.

According to the exemplary embodiments, an order of operations S310 to S350 may be modified, or some operations may be omitted. For example, operations S330 and S340 may be performed in a reverse order. That is, the medical image display apparatus 100 may determine the layout of the medical image list according to the changed size of the first medical image and the size of the display, and change the respective sizes of medical images other than the first medical image in the medical image list according the changed size of the first medical image.

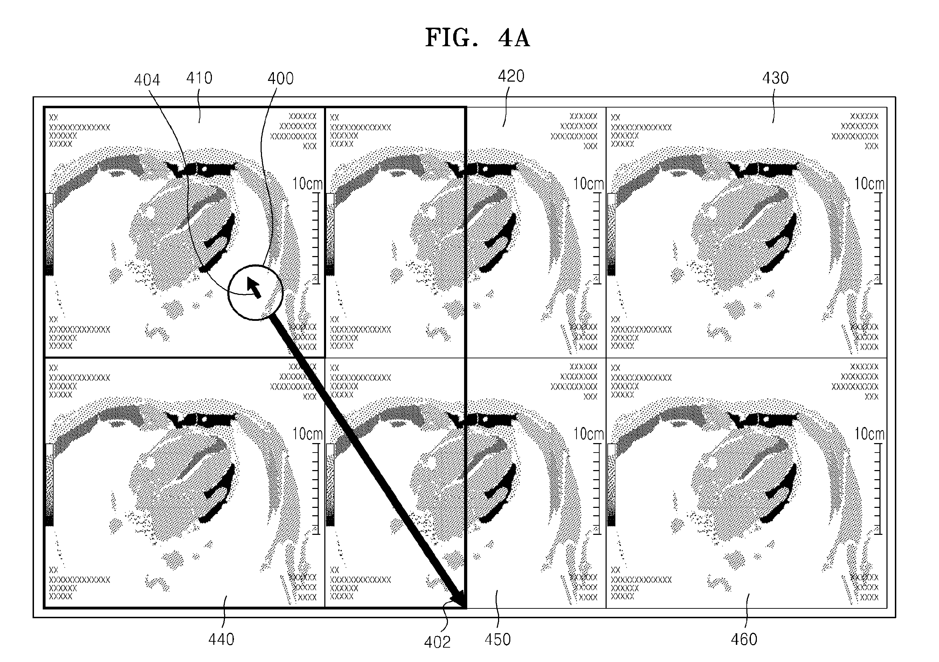

FIGS. 4A to 4C illustrate examples in which the layout of the medical image list changes according to an enlarged size of a first medical image 410.

As illustrated in FIG. 4A, according to an exemplary embodiment, the medical image display apparatus 100 may display a medical image list including the first medical image to a sixth medical image 410, 420, 430, 440, 450, and 460 on the display.

The medical image display apparatus 100 may receive a drag input at an area 400, through a cursor 404, in which a portion of the first medical image 410 is selected and dragged to an area 402 at a lower right side of the display. For example, the portion of the first medical image 410 may be selected by a clicking operation with a mouse.

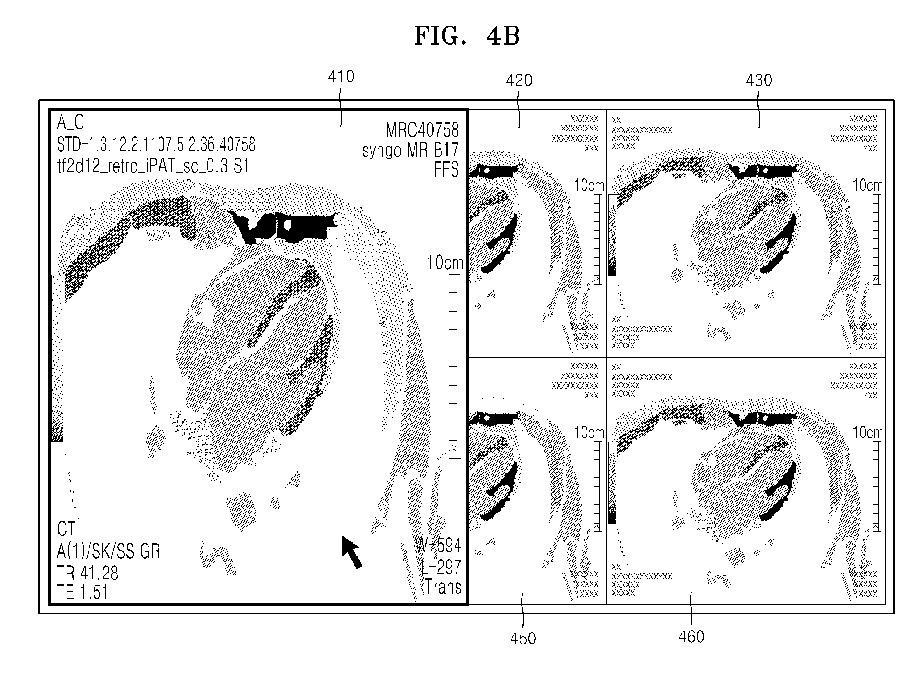

As illustrated in FIG. 4B, according to an exemplary embodiment, the medical image display apparatus 100 may enlarge a size of the first medical image 410 according to at least one of, for example, a direction of the drag input, a length of the drag input, and a finishing location or a time point of the drag input. The medical image display apparatus 100 may increase a size or an amount of a text of the first medical image 410 in response to an increase of the size of the first medical image 410.

Also, in an exemplary embodiment, the medical image display apparatus 100 may change respective sizes of a second medical image 420 to the sixth medical image 460 to be substantially the same as the enlarged size of the first medical image 410. The medical image display apparatus 100 may also increase respective sizes and amounts of texts of the second medical image 420 to the sixth medical image 460.

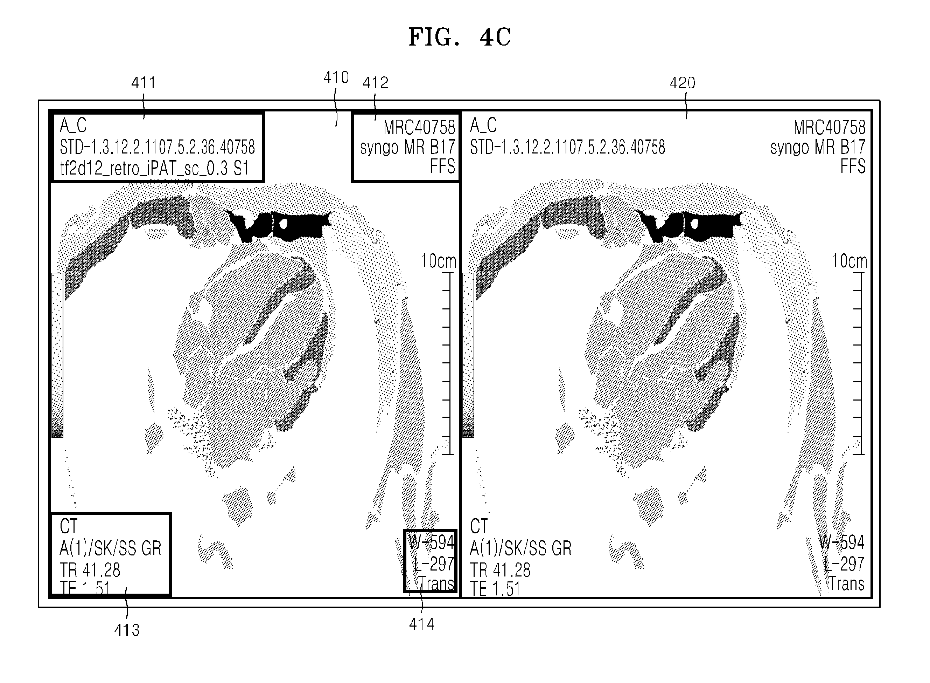

As illustrated in FIG. 4C, the medical image display apparatus 100 may determine a layout of the medical image list according to the changed size of the first medical image 410 and the size of the display, and arrange the medical image list according to the determined layout. For example, since the enlarged first medical image 410 covers substantially one half of the display, the medical image display apparatus 100 may display the first medical image 410 and the second medical image 420 on a current page (i.e., a first page), the third medical image 430 and the fourth medical image 440 on the next page (e.g., a second page), and the fifth medical image 450 and the sixth medical image 460 on the subsequent page (e.g., a third page).

As illustrated in FIG. 4A, when six medical images 410, 420, 430, 440, 450, and 460 are displayed on the display, respective texts of the six medical images 410, 420, 430, 440, 450, and 460 may overlap a portion of the six medical images 410, 420, 430, 440, 450, and 460.

As illustrated in FIG. 4C, when two medical images are displayed on the display, the size of the blank area is increased than when six medical images are displayed. Accordingly, the text of the first medical image 410 (e.g., patient information 411, medical image apparatus information 412, scan environment information 413, and image description information 414) may be displayed in the blank area.

FIGS. 4A to 4C illustrate a case where the user adjusts the size of the first medical image 410 to change the layout of the medical image list. However, the exemplary embodiments are not limited thereto. For example, when the user adjusts a size of the second medical image 420, the medical image display apparatus 100 may change the layout of the medical image list according to the changed size of the second medical image 420.

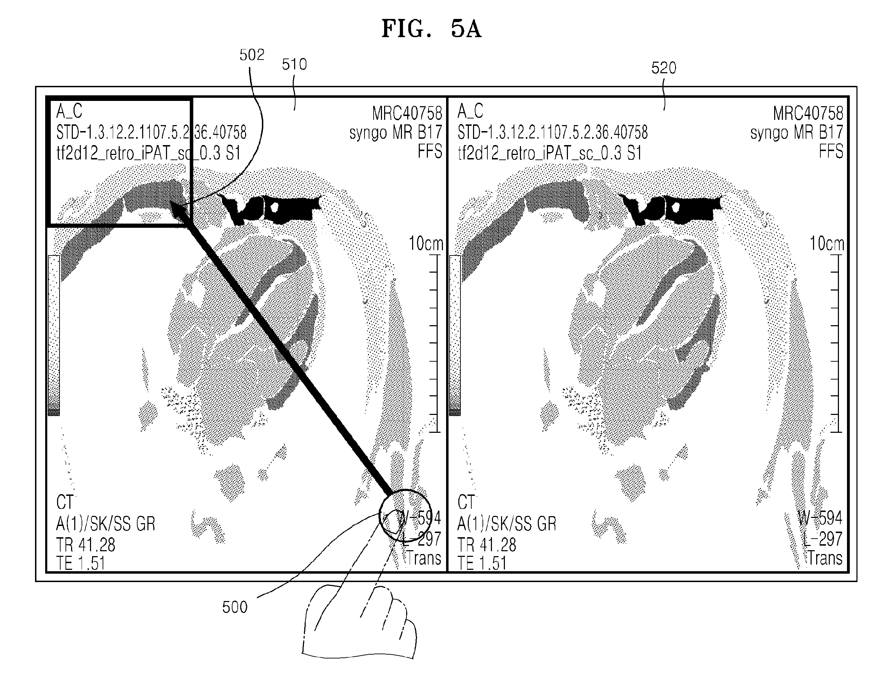

FIGS. 5A to 5C illustrate examples in which the layout of the medical image list is changed according to a reduced size of a first medical image 510.

As illustrated in FIG. 5A, according to an exemplary embodiment, the medical image display apparatus 100 may display a first medical image 510 and a second medical image 520 on the display. The medical image display apparatus 100 may receive a drag input at an area 500 in which a portion of the first medical image 510 is selected and dragged to an area 502 at an upper left side of the display. For example, the portion of the first medical image 510 may be selected by a touch by a finger.

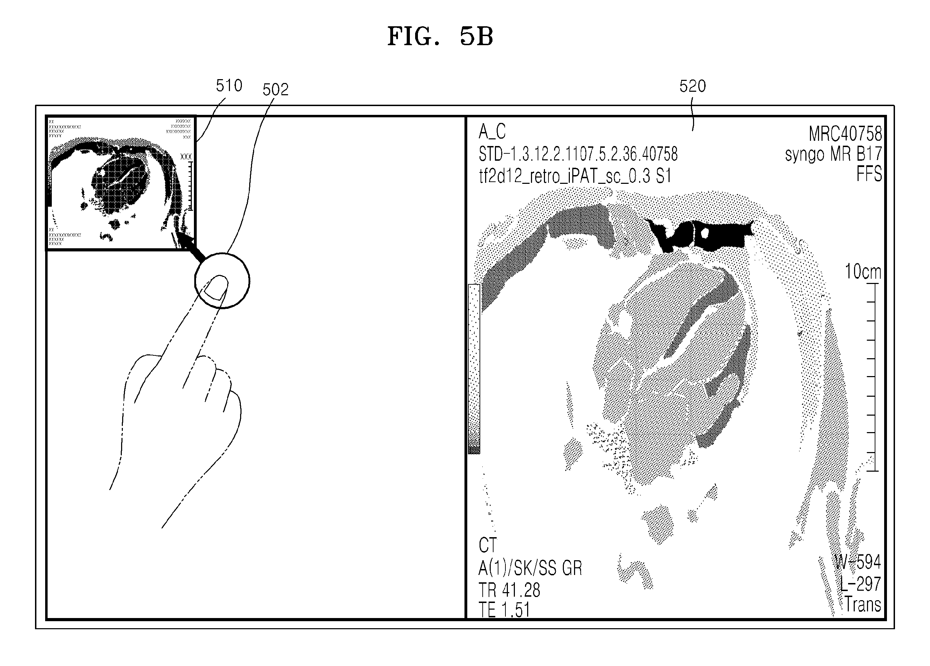

As illustrated in FIG. 5B, according to an exemplary embodiment, the medical image display apparatus 100 may reduce a size of the first medical image 510 according to at least one of, for example, a direction of the drag input, a length of the drag input, and a finishing location or a time point of the drag input. The medical image display apparatus 100 may reduce a size of a text of the first medical image 510 in response to a decrease of the size of the first medical image 510. The medical image display apparatus 100 may also reduce the amount of the text displayed on the display.

Also, the medical image display apparatus 100 may reduce respective sizes of the other medical images (e.g., the second medical image 520) included in the medical image list to be substantially the same as the reduced size of the first medical image 510. The medical image display apparatus 100 may reduce respective sizes or amounts of texts of the other medical images (e.g., the second medical image 520).

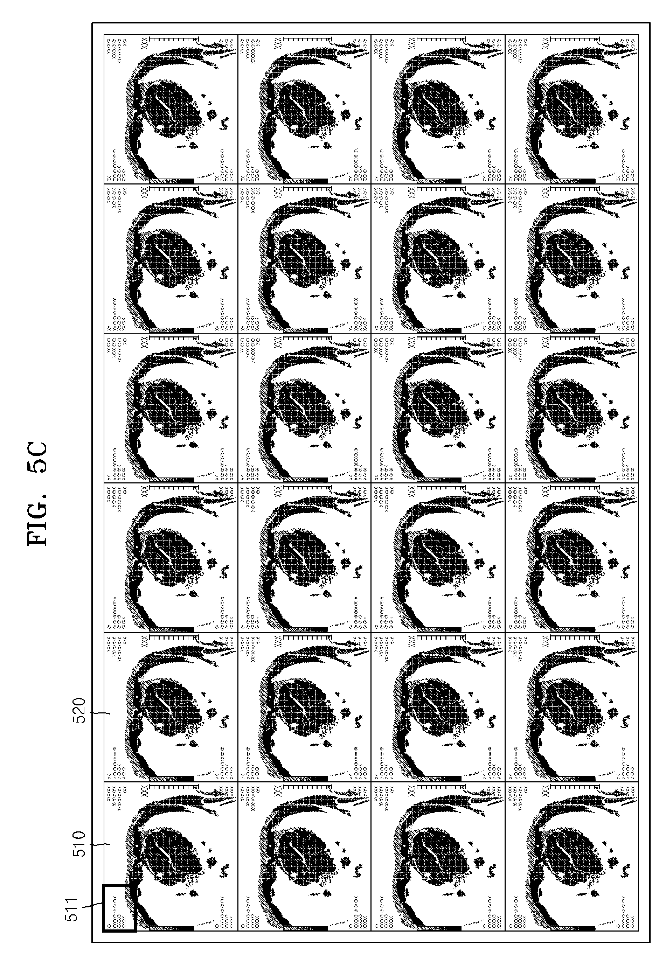

As illustrated in FIG. 5C, the medical image display apparatus 100 may determine the layout of the medical image list according to the changed size of the first medical image 510 and the size of the display, and arrange the medical image list according to the determined layout. For example, since the reduced first medical image 510 covers about 1/24 of the display, the medical image display apparatus 100 may display twenty-four medical images (e.g., the first medical image 510 to a twenty-fourth medical image) having substantially the same size on the display.

As illustrated in FIG. 5A, where two medical images 510 and 520 are displayed on the display, respective texts of the two medical images 510 and 520 may be displayed on the blank area. However, as illustrated in FIG. 5C, when twenty-four medical images are displayed on the display, the size of the blank area is smaller than when the two medical image 510 and 520 are displayed. Accordingly, the text of the first medical image 510 (e.g., patient information 511) may overlap a portion of the first medical image 510.

FIGS. 6A to 6C are views illustrating adjusting a size of at least one medical image included in the medical image list in the medical image display apparatus 100 according to an exemplary embodiment.

According to an exemplary embodiment, the medical image display apparatus 100 may adjust the changed size of the first medical image and the respective sizes of the other medical images according to the determined number of medical images.

As illustrated in FIG. 6A, the medical image display apparatus 100 may change a size of a first medical image 610 according to a user input. Also, the medical image display apparatus 100 may determine the number of medical images to be displayed on an area 600 of the display according to the changed size of the first medical image 610 and the size of the display.

For example, when the size of the display is 12"x12", and the changed size of the first medical image 610 is 5"x3", the medical image display apparatus 100 may determine to display six medical images on the display. When the medical image display apparatus 100 displays the first medical image to a sixth medical image 610, 620, 630, 640, 650, and 660 having a size of 5"x3" on the display, at least a portion of the display may include a blank area 601.

Therefore, the medical image display apparatus 100 may adjust respective sizes of the first medical image 610 to the sixth medical image 660 such that the first medical image 610 to the sixth medical image 660 may be displayed on an entire area of the display. For example, the medical image display apparatus 100 may adjust the respective sizes of the first medical image 610 to the sixth medical image 660 to be 6"x4".

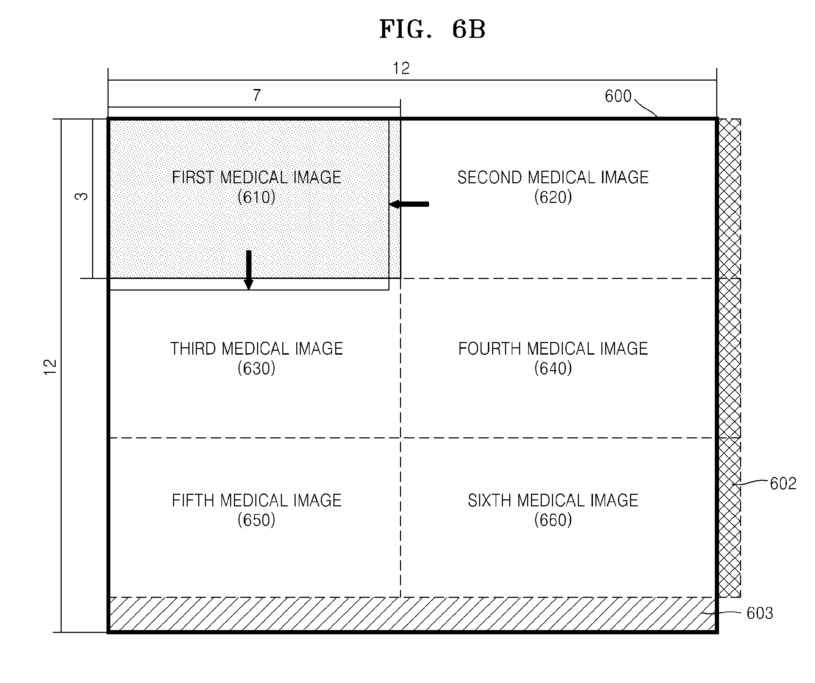

As illustrated in FIG. 6B, the medical image display apparatus 100 may change the size of the first medical image 610 to be 7"x3" according to the user input. Also, the medical image display apparatus 100 may determine the number of medical images to be displayed on the display according to the changed size of the first medical image 610 and the size of the display.

For example, when the size of the display is 12"x12" and the changed size of the first medical image 610 is 7"x3", the medical image display apparatus 100 may determine to display 6 medical images on the display. When the medical image display apparatus 100 displays the first medical image 610 to the sixth medical image 660 having a size of 7"x3" on the display, portions 602 of a second medical image 620, a fourth medical image 640, and the sixth medical image 660 may not be displayed on the display. Also, a portion of the display may include a blank area 603.

Therefore, the medical image display apparatus 100 may adjust the respective sizes of the first medical image 610 to the sixth medical image 660 such that the first medical image 610 to the sixth medical image 660 may be substantially exactly displayed on an entire area of the display. For example, the medical image display apparatus 100 may adjust the respective sizes of the first medical image 610 to the sixth medical image 660 to be 6"x4".

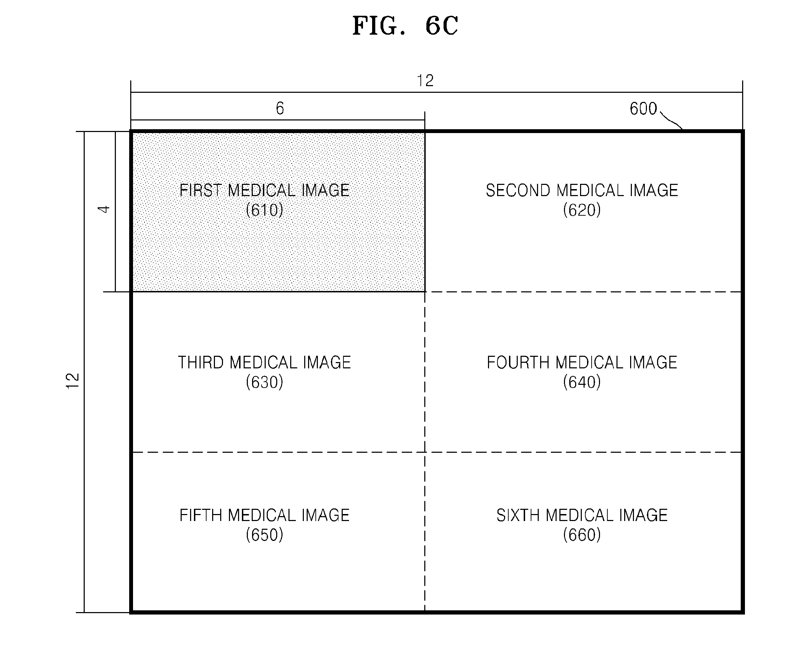

As illustrated in FIG. 6C, when the medical image display apparatus 100 adjusts the respective sizes of the first medical image 610 to the sixth medical image 660 to be 6"x4", the first medical image 610 to the sixth medical image 660 may be substantially uniformly displayed on the display.

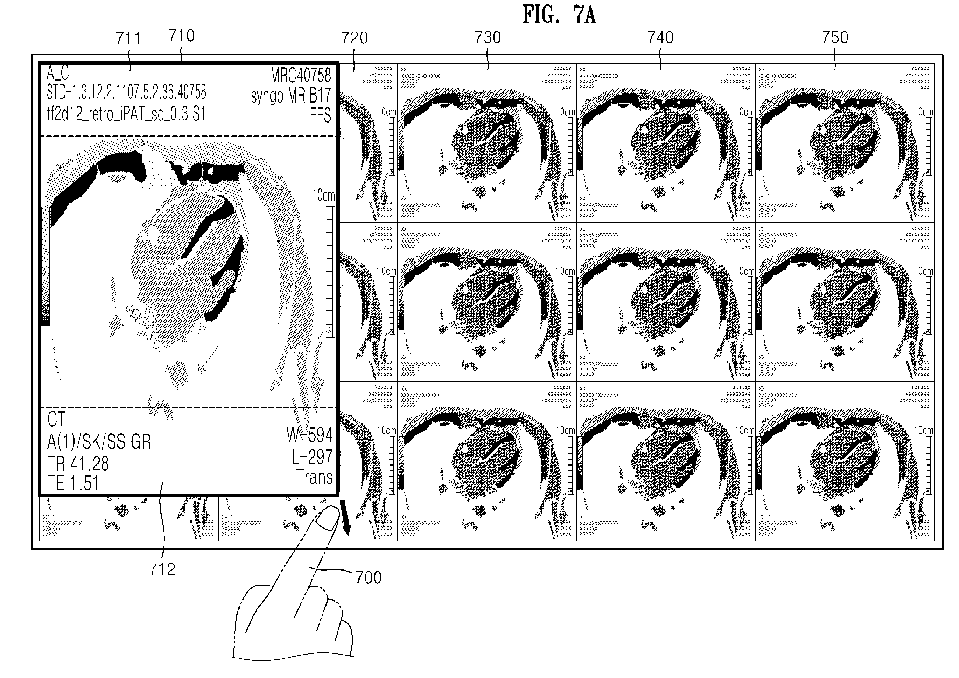

FIGS. 7A to 7D are views illustrating examples of changing the layout of the medical image list according to an aspect ratio of a first medical image 710.

As illustrated in FIG. 7A, the medical image display apparatus 100 may change a size of the first medical image 710 such that a width increase rate is greater than a height increase rate, according to a drag input 700 of the user. That is, according to the user's drag input 700, the aspect ratio of the first medical image 710 may be reduced. In this case, respective sizes (or respective aspect ratios) of the other medical images included in a medical image list, i.e., second to fifth medical images 720, 730, 740, and 750, may be changed to be substantially the same as the changed size (or the changed aspect ratio) of the first medical image 710.

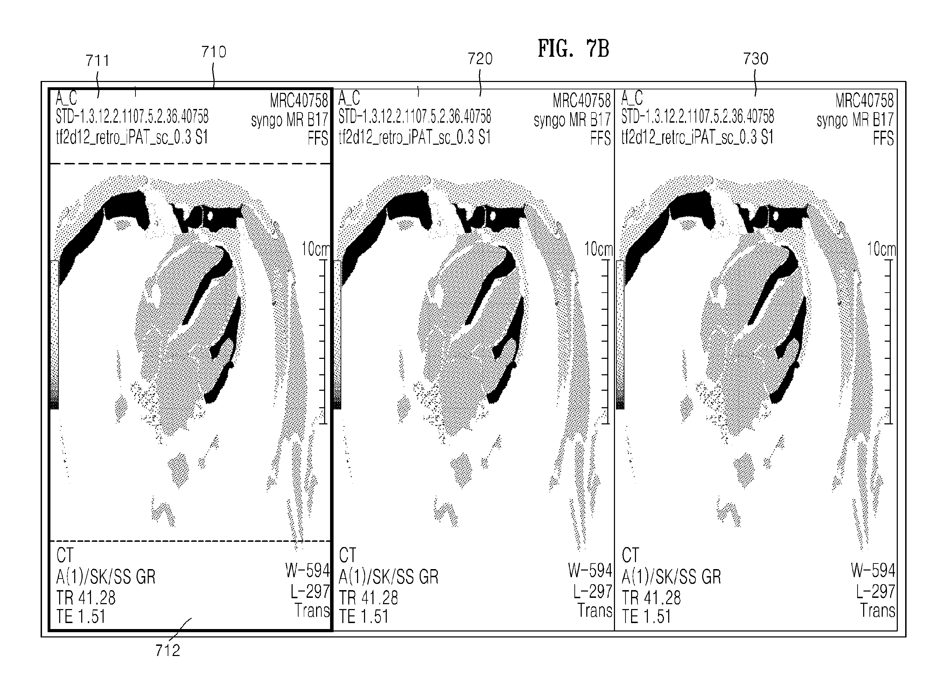

As illustrated in FIG. 7B, the medical image display apparatus 100 may determine the layout of the medical image list according to the changed size (or the changed aspect ratio) of the first medical image 710 and the size of the display. Then, the medical image display apparatus 100 may arrange the medical image list according to the determined layout. For example, when the first medical image 710 having a vertically elongated shape covers substantially one third of the display, the medical image display apparatus 100 may display the first to third medical images 710, 720, and 730 in the vertically elongated shape on the display. Also, the medical image display apparatus 100 may display the fourth medical image 740 to a fifteenth medical image on other pages.

According to an exemplary embodiment, since the first to third medical images 710, 720, and 730 are vertically elongated, upper and lower blank areas 711 and 712 may be increased in size. Therefore, the medical image display apparatus 100 may display a text of the first medical image 710 in the upper and lower blank areas 711 and 712.

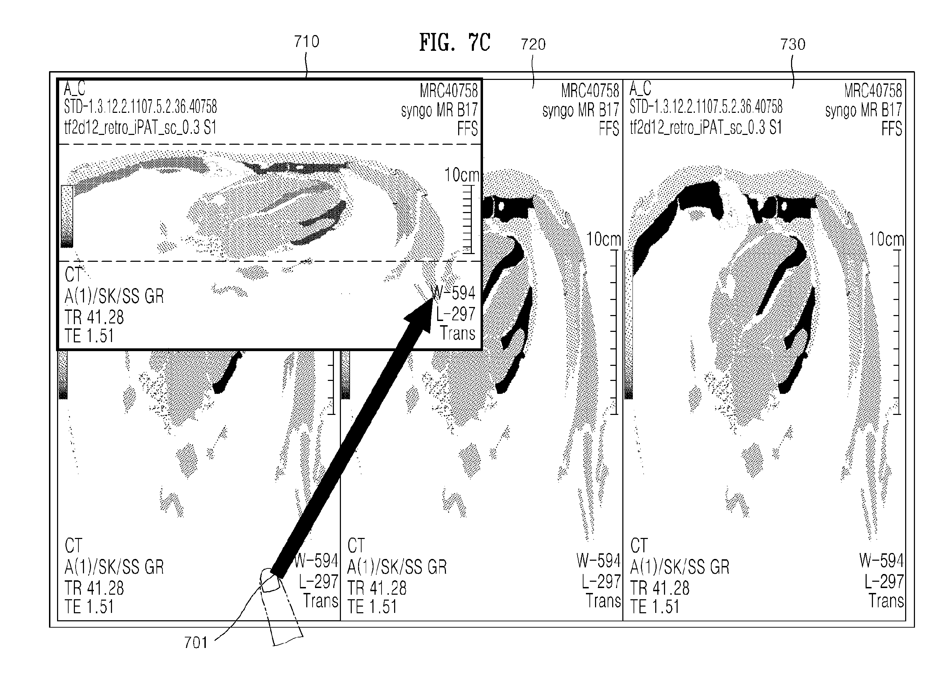

As illustrated in FIG. 7C, the medical image display apparatus 100 may change the size of the first medical image 710 such that the width thereof increases and the height thereof reduces according to a user's drag input 701. That is, according to the user's drag input 701, the aspect ratio of the first medical image 710 may be increased. In this case, the respective sizes (or the respective aspect ratios) of the other medical images included in the medical image list, i.e., the second to fifth medical images 720, 730, 740, and 750, may be changed to be substantially the same as the changed size (or the changed aspect ratio) of the first medical image 710.

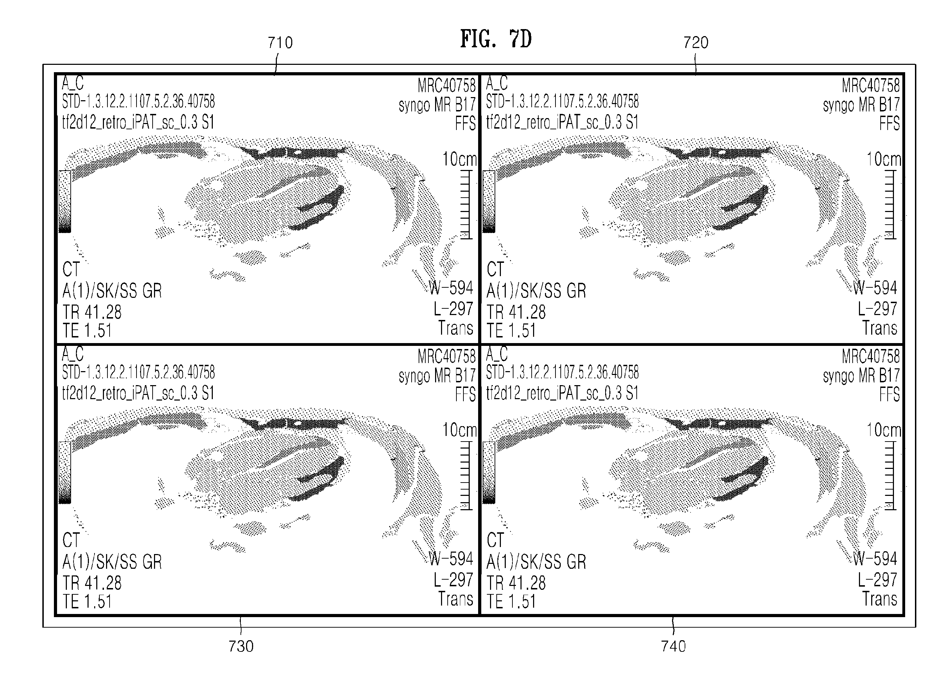

As illustrated in FIG. 7D, the medical image display apparatus 100 may determine the layout of the medical image list according to the changed size (or the changed aspect ratio) of the first medical image 710 and the size of the display. Then, the medical image display apparatus 100 may arrange the medical image list according to the determined layout. For example, when the first medical image 710 having a horizontally longer shape covers substantially one fourth of the display, the medical image display apparatus 100 may display the first to fourth medical images 710, 720, 730, and 740 in the horizontally longer shape on the display. The medical image display apparatus 100 may display the fifth medical image 750 to the fifteenth medical image on other pages.

According to an exemplary embodiment, since the first to fourth medical images 710, 720, 730, and 740 are horizontally longer, the medical image display apparatus 100 may display a text that respectively corresponds to the medical images in a blank area in the horizontally longer shape.

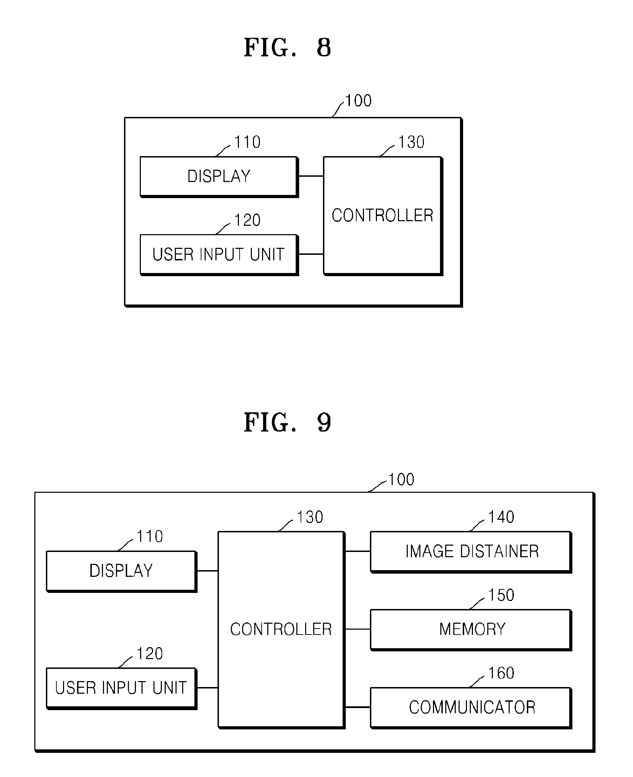

FIGS. 8 and 9 are block diagrams of the medical image display apparatus 100 according to an exemplary embodiment.

As illustrated in FIG. 8, the medical image display apparatus 100 according to an exemplary embodiment may include a display or displays 110, a user input unit 120, and a controller 130. However, it should be noted some of the above elements may not be essential to the exemplary embodiment. Also, the medical image display apparatus 100 may include more or less elements than the above elements. For example, as illustrated in FIG. 9, the medical image display apparatus 100 according to an exemplary embodiment may further include an image obtainer 140, a memory 150, and a communicator 160.

Hereinafter, the elements of the medical image display apparatus 100 will be described in detail.

The display 110 displays information that is processed in the medical image display apparatus 100. For example, the display 110 may display medical images, or a user interface (UI) or a GUI for controlling the medical image display apparatus 100.

The display 110 may display a medical image list that includes at least one medical image, and at least one medical image of the medical image list may be changed in size according to a user input.

In an exemplary embodiment, the display 110 may be provided with a touchpad to form a layer structure and thus configure a touch screen. In this case, the display 110 may be used as an output device as well as an input device. The display 110 may include at least one of, for example, a liquid crystal display (LCD), a thin film transistor (TFT) LCD, organic light-emitting diode (OLED) display, a flexible display, a 3D display, and an electrophoretic display. Also, the medical image display apparatus 100 may include two or more displays 110 according to the exemplary embodiments.

User input data for controlling the medical image display apparatus 100 are received through the user input unit 120. For example, the user input unit 120 may include, but is not limited to, a key pad, a dome switch, a touch pad, a trackball, or a jog switch. When the user input unit 120 is a touch pad, the touch pad may include, for example, a capacitive type, a resistive type, an infrared beam type, a surface acoustic wave type, an integral strain gauge type, a piezoelectric type, etc. For example, the user input unit 120 may further include various input devices such as an electrocardiography module, a respiration measurement module, a voice detector, a gesture detector, a finger scanner, an iris scanner, a depth sensor, or a distance sensor.

The user input unit 120 may receive a user input for changing the size of the first medical image included in the medical image list. For example, the user input unit 120 may receive an input for selecting a predetermined area of the first medical image and a drag input with respect to the selected predetermined area.

The controller 130 controls overall operations of the medical image display apparatus 100. For example, the controller 130 may control the display 110, the user input unit 120, the image obtainer 140, the memory 150, and the communicator 160. For example, the controller 130 may include a central processing unit (CPU).

According to an exemplary embodiment, the controller 130 may change the size of the first medical image according to the user input. For example, the controller 130 may adjust the respective sizes of the other medical images to be substantially the same as the size of the first medical image.

The controller 130 may change an aspect ratio of the first medical image and a size of a text of the first medical image according to the user input.

The controller 130 may change the respective sizes of medical images other than the first medical image in the medical image list according to the changed size of the first medical image. The controller 130 may determine the layout of the medical image list according to the changed size of the first medical image and a size of the display 110. For example, the controller 130 may display the number of medical images to be displayed on the display 110 according to the changed size of the first medical image and a size of the display 110. Also, the controller 130 may determine the number of pages of the medical image list according to the determined number of medical images.

The controller 130 may adjust the changed size of the first medical image and the respective sizes of the other medical images according to the determined number of medical images. The controller 130 may adjust a size of a blank area of the display 110 according to the changed size and the aspect ratio of the first medical image.

The controller 130 may determine a display location of the text of the first medical image according to the changed size of the first medical image and the size of the display 110. Also, the controller 130 may adjust the amount of the text displayed on the display 110.

According to an exemplary embodiment, the controller 130 may arrange the medical image list according to the determined layout.

The image obtainer 140 may acquire medical images of an object. For example, the image obtainer 140 may transmit ultrasound signals from a surface of the object (e.g., a human body) to a predetermined part of the object, and may use ultrasound signals that are reflected from tissues in the predetermined part of the object so as to acquire ultrasound images of the object.

Alternatively, the image obtainer 140 may acquire a sectional image (e.g., an MR image) of a part of the object by expressing, in a contrast comparison, a strength of a MR signal with respect to a RF signal generated in a magnetic field having a specific strength. Alternatively, the image obtainer 140 may acquire a CT image or an X-ray image by irradiating X-rays on the object.

The memory 150 may store programs to be processed by the controller 130, or input and/or output data (e.g., medical image data, subject information, probe information, and body markers).

The memory 150 may include at least one of a flash memory type memory, a hard disk type memory, a multimedia card micro type memory, a card type memory (e.g., secure digital (SD) or an xD memory), a random access memory (RAM), a static RAM (SRAM), a read-only memory (ROM), an electrically erasable programmable ROM (EEPROM), a programmable ROM (PROM), a magnetic memory, a magnetic disk, and an optical disk. Alternatively, the medical image display apparatus 100 may use a web storage or a cloud server that performs a storage function of the memory 150 on the Web.

The communicator 160 may include at least one component to enable communication between the medical image display apparatus 100 and a server or an external device including, for example, a local area communication module, a wired communication module, or a mobile communication module.

The local area communication module may be used to perform local area communication with an external device located within a predetermined distance. Examples of a local area communication technology include Wi-Fi, Bluetooth, BLE, ultra wideband (UWB), ZigBee, near field communication (NFC), Wi-Fi direct (WFD), and infrared data association (IrDA).

The wired communication module may be used to perform communication by using electric signals or optical signals. Examples of a wired communication technology include wired communication technologies using a pair cable, a coaxial cable, an optical fiber cable, and an Ethernet cable.

The mobile communication module transmits or receives wireless signals to or from at least one of a base station, an external device, and a server, in a mobile communication network. The wireless signals may include data in any one of various formats according to transmission and reception of, for example, voice call signals, video call signals, and text and/or multimedia messages.

The communicator 160 wired or wirelessly connected to a network and thus communicates with the external device. The communicator 160 may transmit or receive medical image data to or from a hospital server or another medical apparatus in a hospital connected through a PACS, and perform data communication according to the DICOM standard.

The communicator 160 may transmit or receive data (e.g., medical image data of the object) related to the diagnosis of an object via a network, and may also transmit or receive medical images captured by other medical apparatuses. Furthermore, the communicator 160 may receive information regarding a diagnosis history or a treatment schedule of a patient from a server, and use the information for the diagnosis of the object.

According to an exemplary embodiment, a layout of a medical image list in the medical image display apparatus 100 may be easily changed when the user simply changes a size of a medical image included in the medical image list.

In addition, the exemplary embodiments may also be implemented through computer-readable code and/or instructions on a medium, e.g., a computer-readable medium, to control at least one processing element to implement any above-described embodiments. The medium may correspond to any medium or media which may serve as a storage and/or perform transmission of the computer-readable code.

The computer-readable code may be recorded and/or transferred on a medium in a variety of ways, and examples of the medium include recording media, such as magnetic storage media (e.g., ROM, floppy disks, hard disks, etc.) and optical recording media (e.g., compact disc read only memories (CD-ROMs) or digital versatile discs (DVDs)), and transmission media such as Internet transmission media. Thus, the medium may have a structure suitable for storing or carrying a signal or information, such as a device carrying a bitstream according to one or more exemplary embodiments. The medium may also be on a distributed network, so that the computer-readable code is stored and/or transferred on the medium and executed in a distributed fashion. Furthermore, the processing element may include a processor or a computer processor, and the processing element may be distributed and/or included in a single device.

The foregoing exemplary embodiments and advantages are merely exemplary and are not to be construed as limiting. The present teaching can be readily applied to other types of apparatuses. Also, the description of the exemplary embodiments is intended to be illustrative, and not to limit the scope of the claims, and many alternatives, modifications, and variations will be apparent to those skilled in the art.

## Claims

1. A method of displaying medical images, the method comprising:
displaying a medical image list on a display;
changing a size of a first medical image included in the medical image list according to a user input;
changing respective sizes of other medical images other than the first medical image in the medical image list according to the changed size of the first medical image;
determining a layout of the medical image list according to the changed size of the first medical image and a size of the display; and
aligning the medical image list according to the determined layout.

2. The method of claim 1, wherein the changing the size of the first medical image comprises receiving a drag input at an area of the first medical image.

3. The method of claim 1 or 2, wherein the changing the respective sizes of the other medical images comprises adjusting the respective sizes of the other medical images to be substantially the same as the changed size of the first medical image.

4. The method of claim 1, wherein the changing the size of the first medical image comprises changing an aspect ratio of the first medical image according to the user input.

5. The method of claim 1, wherein the changing the size of the first medical image comprises changing a size of a character included in a text associated with the first medical image.

6. The method of claim 5, wherein the text associated with the first medical image comprises at least one of patient information, information regarding a medical image apparatus in which the first medical image is obtained, scan environment information of the first medical image, a description of the first medical image, and an annotation.

7. The method of any of the previous claims, wherein the determining the layout comprises determining a number of medical images to be displayed on the display according to the changed size of the first medical image and the size of the display.

8. The method of claim 7, further comprising adjusting the changed size of the first medical image and the respective sizes of the other medical images according to the determined number of medical images.

9. The method of claim 7 or 8, wherein the determining the layout comprises determining a number of pages of the medical image list according to the determined number of medical images.

10. The method of claim 4, wherein the determining the layout comprises determining a size of a blank area according to the changed size and the changed aspect ratio of the first medical image.

11. The method of any of the previous claims, wherein the determining the layout comprises determining a display location of a text associated with the first medical image.

12. The method of any of the previous claims, wherein the determining the layout comprises adjusting an amount of a text displayed on the display.

13. An apparatus for displaying medical images, the apparatus comprising:
a display configured to display a medical image list;
a user input unit configured to receive a user input for changing a size of a first medical image included in the medical image list; and
a controller configured to change the size of the first medical image according to the user input, change respective sizes of other medical images other than the first medical image in the medical image list according to the changed size of the first medical image, determine a layout of the medical image list according to the changed size of the first medical image and a size of the display, and aligning the medical image list according to the determined layout.

14. The apparatus of claim 13, wherein the controller is configured to determine a number of medical images to be displayed on the display, according to the changed size of the first medical image and the size of the display.

15. A non-transitory computer-readable recording medium having recorded thereon a program which, when executed by a computer, causes the computer to execute the method of any of the claims 1-12.
